# EUROPEAN PATENT SPECIFICATION

(11) **EP 0 373 782 B1**
(45) Date of publication and mention of the grant of the patent: **05.10.1994**
(21) Application number: 89312245.7
(22) Date of filing: 24.11.1989
(51) Int. Cl.: A61F 5/448

(54) **Ostomy Coupling**
Ostomiekupplung
Accouplement d'ostomie

(30) Priority: 13.12.1988 GB 8829058
(43) Date of publication of application: 20.06.1990
(73) Proprietor: E.R. Squibb & Sons, Inc., Princeton, N.J. 08540-4000 (US)
(72) Inventor: Wiltshire, Neil Philip, Lingfield Surrey (GB); Steer, Peter Leslie, East Grinstead Sussex (GB)
(74) Representative: Cook, Anthony John

(56) References cited:
- EP-A- 286 501
- EP-A- 0 255 310
- "Biotrol System the event" Biotrol pharma

## Description

This invention relates to a coupling for releasably connecting a bag or pouch (for receiving discharged waste) to a pad attached to the body of a wearer. Such bags are often called ostomy bags and such couplings are often called ostomy couplings.

It has been an aim of persons in the art to design a coupling which is simple and economical to manufacture, comprises a minimum number of parts and provides a secure and leak-proof attachment. A successful prior design of a two-part ostomy coupling is shown in British Patent No. 1,571,657. Other designs which are preferable from the points of view of flexibility and reduced thickness are shown in British Patent No. 2,121,902 and British Patent Publication No. 2,193,098 discloses a three-part ostomy coupling which comprises a first part having a flange, a central chute, and an array of spaced projections; a second part having a peripheral seal for engaging and surrounding the outer wall of the chute and an outwardly projecting rim capable of a snap fit with the spaced projections; and a third part which is rotatable to engage the projections in order to effect a positive lock between the rim and the projections. That is to say, the first and second part form the essential sealing and coupling elements between the bag and the body, and the third part is a locking ring which provides a relatively secure locking mechanism to the coupling.

The present invention is an improvement on the ostomy coupling disclosed in British Patent No. 2,193,098 and in this respect it is the aim of the present invention to improve the coupling security between the three interengaging elements. That is to say, when in use, the bag is securely attached to the pad on the body. However, when the bag needs to be removed, this can be achieved manually using a relatively small uncoupling force without adversely affecting its leakproof quality.

There has been proposed, in European Patent Application No. 286501A1, which was published on 12 October 1988, an ostomy appliance, referred to as a safety device for stoma apparatus, comprising two elements, one of which is adapted to be fixed about an artificial opening in the body of the user by means of a suitable fixing means such as an adhesive medium which is sensitive to pressure, a belt or any other similar system, while the other is rigid with a bag for collecting body fluids and/or waste intended to be assembled in detachable fashion on the said first element by socket-type fitment under the action of a pressure which is exerted as the two aforesaid elements are brought close to each other, the said device being characterised in that the element associated with the pad forming a protection to the skin on the peristomal area and around the orifice of one or more probes or the like with which it is rigid comprises, about a first tubular socket, a rotating device for locking engagement, in respect of the said first socket, of a second socket associated with the bag and capable of being locked in respect of the first socket when the two sockets co-operate in a socket-type attachment by resiliently deformable click-stop means provided on the first socket and of a discreet number. This European Patent Application appears to relate to the product which is described and illustrated in the document entitled "Biotrol System The Event" which apparently was published by Biotrol Pharma of 1 Rue de Foin, 75140 Paris Cedex 03, France. It is asserted that this document was published in June 1988.

According to the present invention there is provided a three part ostomy coupling which has a first part having a flange and a central chute for surrounding the stomal opening; a second part having a peripheral seal for engaging and surrounding the outer wall of the chute; and a third part in the form of a locking ring which is rotatable relative to the first part in order to effect a positive lock between the first and second parts; the coupling being arranged so that rotation of the locking ring to one position prevents the disengagement of the first and second parts of the coupling and to another position allows the parts to be sprung apart manually; said first part also having a plurality of spaced members arranged in a substantially circular array surrounding the central chute, these spaced members each having outwardly and inwardly projecting portions; characterised by the spaced members being arcuate walls whose outwardly and inwardly projecting portions are flanges; by the second part having a radially-outwardly projecting rim capable of a snap fit with the arcuate walls; and by the locking ring having on its upper surface ridge-like projections which extend in an axial direction from the ring and engage under the outwardly projecting flanges on the arcuate walls on rotation of the ring to effect the locking of the first and second coupling parts, the ridge-like projections of the locking ring being operative to deform the outwardly projecting flanges of the arcuate walls such that the inwardly projecting flanges overlap the said rim.

In use, rotation of the locking ring to one position prevents the disengagement of the two parts of the coupling, and to another position allows the parts to be sprung apart manually.

The invention will be more clearly understood from the following non-limiting description of an example thereof given with reference to the accompanying drawings in which:
Figure 1 illustrates in axial cross-section one example of a first part which is preferably but not necessarily a body side coupling part or element;
Figure 2 illustrates in axial cross-section one example of a second part which is preferably but not necessarily a bag side coupling part or element;
Figure 3 illustrates in axial cross-section one example of a third part, the locking ring; the common axis 16 of the stomal aperture 18 being shown in these Figures as a vertical chain dotted line;
Figure 4 is a diagrammatic front view of the third part, the locking ring;
Figure 5 illustrates in axial cross-section the assembled coupling;
Figure 6 is a diagrammatic front view of the second part, of Figure 2; and
Figure 7 is a diagrammatic back view of the second part of Figure 2.

The illustrated ostomy coupling includes a first coupling part or element 20, preferably but not necessarily the body-side coupling element, a second coupling part or element 10, preferably but not necessarily the bag-side coupling element, and a locking ring 30.

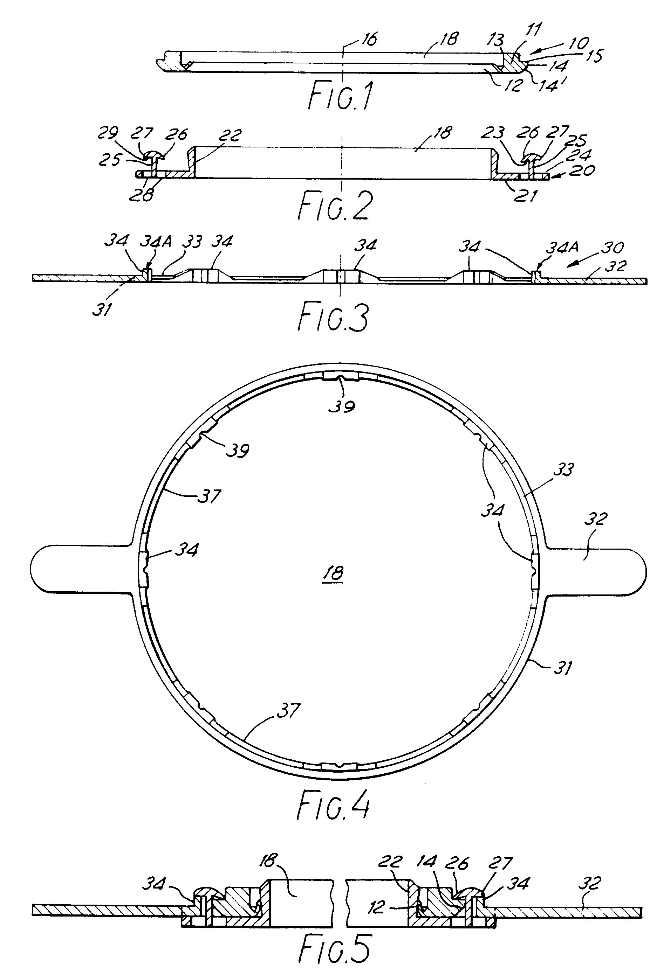

The second coupling part 10 illustrated in Figure 1 is the bag-side coupling element in this embodiment of the invention. It includes a side member 11 encircling the aperture 18, the rib member 11 being integral with a deflectible, peripheral seal 12 located on the radially inner side of the rib member 11 and with a rim 14 located on the radially outer side of the rib member 11. The second coupling element 10 is mounted to the bag wall on the annular bottom surface 13 of the rib member 11 by heat sealing, adhesive, etc. The seal 12 extends back toward the plane of the bottom surface 13 at an acute angle to the rib member 11. The rim 14 is in part defined by a surface 15 which lies in a plane normal to the axis 16, although of course a surface located in plane at a small angle to the normal could also be employed.

The bag-side coupling element 10 may also include a filter housing (not shown). In British Patent Application Publication No. 2,177,926, there is described a bag-side coupling element for an ostomy bag, the element having a flange surrounding a stomal orifice and having a coupling portion by means of which it can be coupled to a body side coupling element, characterised by the flange having an extension at an upper part thereof, (i.e. towards the top of the bag when the element is connected thereto) the extension including a flat portion having a gas passage hole therein and a peripheral wall attached to the flat portion, the peripheral wall serving to define a filter-receiving recess.

The first coupling part 20 illustrated in Figure 2 is the body side coupling part in this embodiment of the present invention. It comprises a flange 24 whose under surface 21 is intended for attachment in any suitable manner to a pad of medical or surgical adhesive. Made in one piece with the flange 24 is a cylindrical wall 22 constituting a chute for guiding waste material to the interior of a bag or pouch.

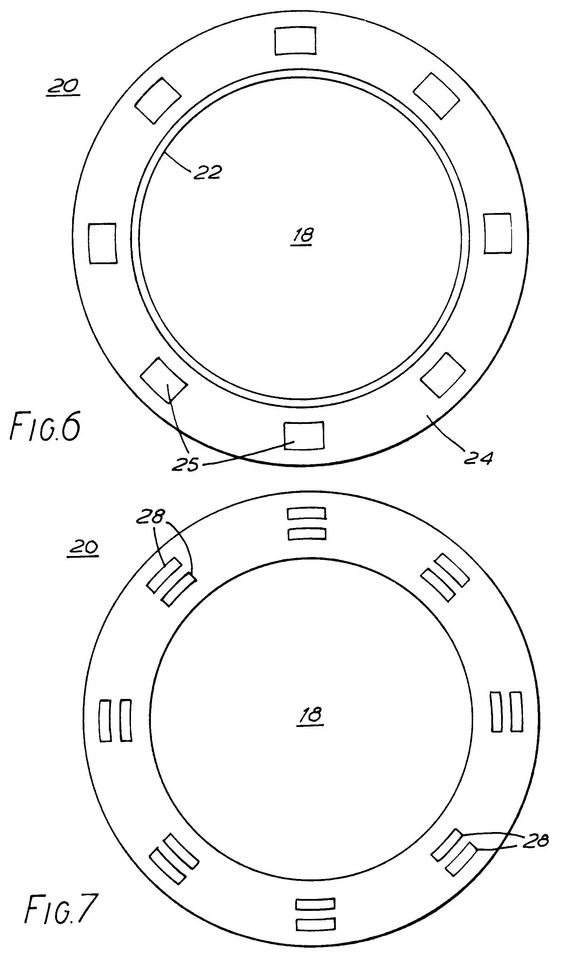

Also forming part of the body side coupling part are a series of arcuate walls 25 arranged to constitute an interrupted encircling wall. Each of the walls 25 has an inwardly projecting flange 26 and an outwardly projecting flange 27. Slots 28 are provided in the flange 24 beneath the flanges 26 and 27 on the arcuate walls 25 to allow a molding tool to be removed when the element 20 is molded. The arcuate walls extend upwardly from an annular region of the flange 24. As seen, the flanges 26 taper toward their radially inward end, this construction being adopted to make them flexible and capable of deformation. In assembling the two coupling parts 10 and 20 together, the parts are moved axially towards each other and the rim 14 on the part 10 is forced past the flanges 26. The outer curved surface 14 engages the outer curved surface on flange 26 to move flange 26 downward and wall 25 outwardly aside the underside of the rim 14. Due to their inherent resilience, these flanges 26 then spring upwardly once the coupling elements are in their relative position illustrated in Figure 5. The elements are coupled together with the annular surface 15 engaging the underside 23 of the flange 26. When the parts 10 and 20 are coupled together the seal 12 deflects and engages the external wall of the chute 22, the two cooperating together to provide a seal. It will be observed that when the two parts 10 and 20 are assembled as shown in Figure 5, the wall 22 prevents any faecal waste material discharged by the wearer and conducted by the chute to the bag interior from having access to, or being able to lodge in, crevices in the other part 10 of the coupling.

The outwardly directed flange 27 of the body side coupling part 20 serve to overlap the locking rin 30 shown in Figures 3 and 4. The locking ring has at least one tab 32 protruding from the outer edge of the ring 31, the purpose of which is to facilitate the rotation and removal of the ring from its position in the ostomy coupling as shown in Figure 5. The upper inner surface of the ring 33 has a number of ridge-like projections 34 (herein also called locking means) which effect a locking of the coupling, and these projections 34 are regularly spaced around the ring 30 such that they subtend equal angles at the axis 16, and also such that they substantially coincide with the number and spacing of the arcuate walls 25.

In assembling the coupling, the locking ring 30 is moved axially toward the body side coupling element, and the edge of the ring 30 is forced past the flanges 27. Due to their inherent resilience, these flanges then spring back once the locking ring is in position.

The locking ring 30 fits around the outside of the arcuate walls 25 adjacent and concentric with those walls. To lock the coupling parts together, the ring 30 is rotated by the wearer using a tab 32 until the ridge-like projections 34 on the upper inner edge of the ring 31 are substantially in registry with the flanges 27 on the body side coupling part 20.

When in a locked position, the top surfaces 34A of the ridge-like projections 34 make face-to-face engagement with the undersurface 29 of the flanges 27 and hence obstruct the walls 25 from moving radially outwardly. The engagement of the top surfaces 34A with the undersides of the flange 27 as seen best in Figure 5 yields a particularly secure lock between the parts of the coupling. Moreover, when the ring is in its unlocked position, the lower profile of the inner portion of the ring 31 in the regions 37 between the projections 34 offer very little obstruction to radially outward movement of the walls 25, which permits the coupling to be separated by a relatively light separating force. As will be understood, coupling separation by a light force is particularly desirable for elderly users.

In other words, the locking means 34 as a whole act in such a way that the body side and bag side coupling parts are held more securely together. However, the locking ring can be rotated out of the "locked" position with relatively little force such that uncoupling can be effected with a relatively small uncoupling force. That is to say in an unlocked position the ring provides less obstruction to the arcuate walls 25 and the flanges 27 such that for disengagement of the body side and bag side coupling parts, the arcuate walls have a much greater flexibility than in the locked position. The bag side and body side coupling parts can thus be separated from each other manually by an axial pull which results in a slight deformation of the projections 25 permitting the rim 14 to spring past the inwardly extending flanges 26 of the respective projection.

The projections 34 include an arcuate cut out 39 which are disposed to mate with protruding pips (not shown) on the radially outer side of the arcuate cut outs 25 beneath outwardly projecting flange 27. This provides a means for registering the arcuate walls with the projections 34 when locking the coupling.

The disclosed and illustrated arrangement provides a positive assurance that the parts of the coupling cannot be separated except when desired by the wearer. On the other hand, due to the ease with which the ring is unlocked, separation of the coupling is achieved with relatively little force and without adversely affecting the leak-proof quality of the coupling seal.

## Claims

1. A three part ostomy coupling which has a first part (20) having a flange (21) and a central chute (22) for surrounding the stomal opening (18); a second part (10) having a peripheral seal for engaging and surrounding the outer wall of the chute (22); and a third part in the form of a locking ring (30) which is rotatable relative to the first part (20) in order to effect a positive lock between the first and second parts (20, 10); the coupling being arranged so that rotation of the locking ring (30) to one position prevents the disengagement of the first and second parts (10, 20) of the coupling and to another position allows the parts to be sprung apart manually; said first part also having a plurality of spaced members arranged in a substantially circular array surrounding the central chute, these spaced members each having outwardly and inwardly projecting portions (26,27); characterised by the spaced members being arcuate walls (25) whose outwardly and inwardly projecting portions are flanges (26,27); by the second part having a radially-outwardly projecting rim (14) capable of a snap fit with the arcuate walls (25); and by the locking ring (30) having on its upper surface ridge-like projections (34) which extend in an axial direction from the ring (30) and engage under the outwardly projecting flanges (27) on the arcuate walls (25) on rotation of the ring (30) to effect the locking of the first and second coupling parts, the ridge-like projections (34) of the locking ring (30) being operative to deform the outwardly projecting flanges (27) of the arcuate walls such that the inwardly projecting flanges (26) overlap the said rim (14).

2. A coupling according to claim 1 in which the said two parts (10, 20) of the coupling are of a resilient plastics material and said second part (10) surrounds the stomal orifice, and said outwardly projecting rim (14) extends therefrom in a direction away from the stomal orifice (18), said plurality of inwardly projecting flanges (26) each having an underside which engages said rim (14) to couple said first and second parts and past which, due to the resilience of the material of the flange (26), the rim (14) can be sprung to separate or couple the two parts (10, 20).

3. A coupling according to claim 1 or claim 2 in which the projections (34) are regularly spaced around the locking ring (30) such that they subtend equal angles at the axis (16) and also such that they substantially coincide in number and spacing with the arcuate walls (25), in the locked condition of the coupling parts (10, 20).

## Patentansprüche

1. Dreiteilige Ostomiekupplung, die ein erstes Teil (20) mit einem Flansch (21) und einer zentralen Rutsche (22) zum Umgeben der Stomaöffnung (18), ein zweites Teil (10) mit einer Umfangsdichtung zum in Eingriff nehmen und umgeben der Außenwand der Rutsche (22), und ein drittes Teil in Form eines Feststellrings (30) aufweist, der gegenüber dem ersten Teil (20) drehbar ist, wodurch eine positive Verriegelung zwischen dem ersten und zweiten Teil (20, 10) bewirkt wird, wobei die Kupplung so angeordnet ist, daß die Drehung des Feststellrings (30) in eine Stellung das Lösen der zwei Teile (10, 20) der Kupplung verhindert, und in eine andere Stellung ermöglicht, daß die Teile manuell voneinander gelöst werden können, wobei das erste Teil außerdem mehrere in einer im wesentlichen kreisförmigen Anordnung die zentrale Rutsche umgebende beabstandete Elemente aufweist, wobei diese beabstandeten Elemente jeweils nach außen und nach innen vorspringende Abschnitte (26, 27) aufweisen, dadurch gekennzeichnet, daß die beabstandeten Elemente gebogene Wände (25) sind, deren nach außen und innen vorspringende Abschnitte Flansche (26, 27) sind, daß das zweite Teil einen radial nach außen vorspringenden Rand (14) aufweist, der einen Schnappsitz mit den gebogenen Wänden (25) einnehmen kann, und daß der Feststellring (30) an seiner oberen Seite stegartige Vorsprünge (34) aufweist, die sich in einer Axialrichtung von dem Ring (30) erstrecken und unter den nach außen vorspringenden Flanschen (27) an den gebogenen Wänden (25) in Eingriff kommen, wenn der Ring (30) zum Bewirken der Verriegelung der ersten und zweiten Kupplungsteile gedreht wird, wobei die stegartigen Vorsprünge (34) des Feststellrings (30) bewirken, daß die nach außen vorspringenden Flansche (27) der gebogenen Wände so verformt werden, daß die nach innen vorspringenden Flansche (26) den genannten Rand (14) überlappen.

2. Kupplung nach Anspruch 1, wobei die zwei Teile (10, 20) der Kupplung aus einem elastischen Kunststoffmaterial sind und das zweite Teil (10) die Stomaöffnung umgibt, und der nach außen vorstehende Rand (14) sich davon in einer Richtung weg von der Stomaöffnung (18) erstreckt, wobei die mehreren nach innen vorstehenden Flansche (26) jeweils eine Unterseite haben, die mit dem Rand (14) in Eingriff kommen, so daß das erste und zweite Teil verbunden und aufgrund der Elastizität des Materials des Flansches (26) der Rand (14) zum Trennen oder Verbinden der zwei Teile (10, 20) über diese ein- bzw. ausgerastet werden kann.

3. Kupplung nach Anspruch 1 oder Anspruch 2, wobei die Vorsprünge (34) regelmäßig um den Feststellring (30) beabstandet sind, so daß sie an der Achse (16) gleiche Winkel einschließen und so daß sie außerdem im geschlossenen Zustand der Kupplungsteile (10, 20) im wesentlichen in Anzahl und Abstand mit den gebogenen Wänden (25) übereinstimmen.

## Revendications

1. Raccord de stomie en trois parties qui comprend une première partie (20) comportant une bride (21) et une goulotte centrale (22) entourant l'ouverture stomale (18); une seconde partie (10) comportant un joint d'étanchéité périphérique destiné à s'engager sur et à entourer la paroi extérieure de la goulotte (22); et une troisième partie, sous la forme d'une bague de verrouillage (30), qui peut tourner par rapport à la première partie (20) afin de réaliser un verrouillage sûr entre la première et la seconde parties (20, 10); le raccord étant agencé de telle sorte que la rotation de la bague de verrouillage (30) dans une position empêche la séparation de deux parties (10, 20) du raccord, et que sa rotation dans une autre position permet d'écarter élastiquement ces parties, à la main; ladite première partie comportant également plusieurs éléments espacés disposés en une série sensiblement circulaire entourant la goulotte centrale, chacun de ces éléments ayant des parties en saillie vers l'extérieur et vers l'intérieur (26, 27); caractérisé en ce que les éléments espacés sont des parois en arc de cercle (25) dont les parties en saillie vers l'extérieur et vers l'intérieur sont des brides (26, 27); en ce que la seconde partie comporte un rebord (14) faisant saillie radialement vers l'extérieur et pouvant s'encliqueter avec les parois en arc de cercle (25); et en ce que la bague de verrouillage (30) comporte à sa surface supérieure des saillies (34) en forme d'arêtes qui partent axialement de la bague (30) et qui s'engagent sous les brides (27) faisant saillie vers l'extérieur sur les parois en arc de cercle (25) lors de la rotation de la bague (30) pour réaliser le verrouillage de la première et de la seconde parties du raccord, les saillies en forme d'arêtes (34) de la bague de verrouillage (30) ayant pour effet de déformer les brides (27) faisant saillie vers l'extérieur des parois en arc de cercle de telle sorte que les brides (26) faisant saillie vers l'intérieur recouvrent partiellement ledit rebord (14).

2. Raccord selon la revendication 1, dans lequel lesdites deux parties (10, 20) du raccord sont faites d'une matière plastique élastique et ladite seconde partie (10) entoure l'orifice stomal, et ledit rebord (14) faisant saillie vers l'extérieur en part dans une direction opposée à l'orifice stomal (18), lesdites plusieurs brides (26) faisant saillie vers l'intérieur ayant chacune une face inférieure qui s'engage sur ledit rebord (14) pour assembler lesdites première et seconde parties et au-delà de laquelle, sous l'effet de l'élasticité du matériau qui constitue la bride (26), on peut écarter élastiquement le rebord (14) pour séparer ou assembler les deux parties (10, 20).

3. Raccord selon la revendication 1 ou la revendication 2, dans lequel les saillies (34) sont régulièrement réparties autour de la bague de verrouillage (30) de telle sorte qu'elles sous-tendent des angles égaux au niveau de l'axe (16), et aussi de telle sorte qu'elles coïncident sensiblement, en nombre et en espacement, avec les parois en arc de cercle (25), dans la position verrouillée des parties (10, 20) du raccord.
